(19) Europäisches Patentamt
European Patent Office
Office européen des brevets

(11) Publication number: 0 100 366
A1

(12) # EUROPEAN PATENT APPLICATION

published in accordance with Art. 158(3) EPC

(21) Application number: 82900388.8

(22) Date of filing: 09.02.82

Data of the international application taken as a basis:

(86) International application number:
PCT/JP82/00037

(87) International publication number:
WO83/02724 (18.08.83 83/19)

(51) Int. Cl.³: A 61 K 37/54

(43) Date of publication of application:
15.02.84 Bulletin 84/7

(84) Designated Contracting States:
FR

(71) Applicant: MOCHIDA PHARMACEUTICAL CO., LTD.
7, Yotsuya 1-chome
Shinjuku-ku Tokyo 160(JP)

(72) Inventor: OHNISHI, Haruo
4-14 Higashifunabashi 6-chome
Funabashi-shi Chiba 273(JP)

(72) Inventor: KOSUZUME, Hiroshi
1002-10, Honmokucho 4-chome Naka-ku
Yokohama-shi Kanagawa 231(JP)

(72) Inventor: SUZUKI, Yasuo
234-29, Ohaza-Sashima Kawaguchi-shi
Saitama 333(JP)

(72) Inventor: MOCHIDA, Ei
5-4, Komagome 2-chome Toshima-ku
Tokyo 170(JP)

(74) Representative: Moncheny, Michel et al,
c/o Cabinet Lavoix 2 Place d'Estienne d'Orves
F-75441 Paris Cedex 09(FR)

(54) **AGENT FOR TREATING ALLERGIC DISEASE, IMMUNITY COMPLEX DISEASE, AND TUMOUR.**

(57) An agent for treating allergic disease, immunity complex disease, and tumor, containing human pepsin as an effective ingredient. This substance is a protein of human origin, and hence it is a highly safe substance with extremely low side effects such as anaphylactic shock induced by antigenicity of a substance.

FIG.1

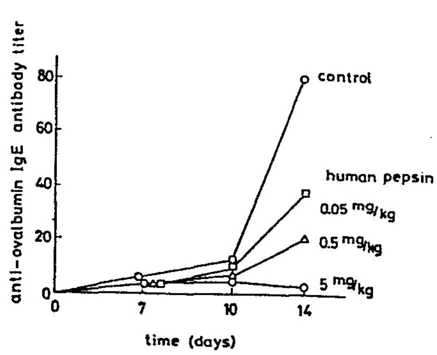


EP 0 100 366 A1

## Technical Field

This invention relates to a therapeutic agent for the treatment of allergic disorders, immune complex diseases and tumors which contains human pepsin as an effective ingredient.

## Background Art

Allergic disorders are caused by an allergic reaction which, as a result of an antigen-antibody reaction, brings about a pathogenesis in a living organism. The mechanism of pathogenesis of allergic disorders is believed to follow the following course. When exposed to a pathogenic antigen, a living organism produces antibodies. The second attack of the same antigen causes an antigen-antibody reaction and, as a result, chemical mediators are released from the cells. These mediators damage tissues and/or the antigen-antibody complexes formed are deposited on the tissues, causing allergic disorders or autoimmune disorders. Among various pathogenic antigens are included xenogenic antigens, such as inhaled allergens, food allergens, drugs, contact allergens, and others such as allogenic or autologus antigens which originate from autologus components of the tissues or organs denatured for some reason and thereby behaving as foreign substances.

So-called allergic disorders caused by xenogenic antigens, such as bronchial asthma, food allergy or urticaria, are classified into four types according to their symptoms or causes.

That is, they are classified into Type I allergies (anaphylactic-type) resulting from tissue-depositing antibodies and characterized by increased capillary permeability and smooth muscle contraction, Type II allergies (cytotoxic-type) resulting in the presence of complements and characterized by cell damage, Type III allergies (Arthus-type) resulting from the deposition of antigen-antibody complexes on vascular walls and subsequent participation of complements and polymorphonuclear leucocytes and characterized by inflammatory reactions, and Type IV allergies resulting from cell-mediated immunity and characterized by the appearance of delayed hypersensitivity such as Tuberculin reaction. Among the allergic reactions, Types I, III and IV allergies participate in, for example, bronchial asthma and each of these reactions is considered independently or in combination to cause asthma attacks. The pathogenic mechanism of these allergic disorders can be considered as follows.

An antigen which invades a living organism is met by macrophages, and the immunological information on the antigen is transmitted to the T cell-B cell system. The B cells which have received the information produce immunoglobulin (Ig E antibody is mainly produced in Type I allergies and Ig G antibody is mainly produced in Type II and Type III allergies) and the Ig E antibody binds to the basophils in the circulation or to the mast cells in the tissues, thereby establishing the state of sensitization. The same antigens upon invading the sensitized organism bind to the cell-bound antibodies, allowing them to release chemical mediators such as histamine and slow-reacting substances of anaphylaxis (SRS-A). The released chemical mediators induce allergic symptoms such as erythema, edema or increase of glandular secretion caused by contraction of smooth muscles and increase of capillary permeability. On the other hand, Ig G antibody binds to polymorphonuclear leucocytes to achieve sensitization and the subsequent secretion of SRS-A as a chemical mediator is also suspected.

Anti-allergic agents may achieve their therapeutic purpose by suppressing any step in these processes.

For instance, xanthine derivatives, β-adrenergic stimulants (β-stimulants) or corticosteroids have been used for the treatment of asthma. However, these drugs are frequently observed to show undesirable adverse reactions. For instance, palpitation, tachycardia, etc., are reported with respect to xanthine derivatives and β-stimulants. Furthermore, corticosteroids cause adverse reactions such as peptic ulcer and complications of bacterial infection. Moreover, anti-histamine agents may cause difficulty in the expectoration of tracheal secretion, rather than being effective against asthma attack, so that they may sometimes worsen the clinical condition of asthma.

Immune complex diseases or autoimmune disorders in which the pathogenic antibody is an auto-antigen, typified by rheumatoid arthritis, systemic lupus erythematosus (SLE) and lupus nephritis, as implied by the names, are disorders resulting from complexes of antigens and antibodies, namely, immune complexes. Although the pathogenic mechanism of immune complex diseases is complicated and has not been resolved in many respects, it is generally believed to follow the following course. When the tissues are damaged by bacterial or viral infections, antibodies are produced against the freshly produced auto-antigens or virally-infected cells and they react with the corresponding antigens to form immune complexes. Since these immune complexes activate the complement system and platelets, vasoactive substances such as histamine and serotonin are released, so that the capillary permeability is increased. Then, the immune complexes in circulation penetrate into the vascular walls having increased permeability and deposit along the basement membranes. Polymorphonuclear leucocytes are gathered on the deposited sites of the immune complexes by the action of the leucocyte chemotactic factors produced by the action of the complement upon the deposited immune complexes. The polymorphonuclear leucocytes, reacting with the immune complexes, release various tissue-damaging substances such as cathepsin D and E, collagenase, elastase and permeability factors, and these substances eventually damage the tissue. The level of complement in the serum from a patient with an immune complex disease such as SLE is generally low and aggravation of the disease conditions is closely correlated with the decrease of the complement level. This decline of complement level is considered to be due to a plentiful consumption of complement at the site where the reaction between antigens and antibodies takes place, such as in the kidneys and blood vessels. Moreover, the immune complexes also appear to be related to blood coagulation systems and they are believed to lead to more serious conditions through diverse mechanisms such as the acceleration of fibrinoid deposition onto the damaged tissues.

For the treatment of immune complex diseases, antiinflammatory agents and immunosuppressive agents including steroids are presently used for suppressing the hypersensitized immune system and for reducing local inflammations and pains, or anticoagulants and antiplatelet agents are used for improving abnormalities of the coagulation-fibrinolysis system in the blood vessels. However, because these drugs show weak effectiveness and are associated with strong adverse reactions, it has been strongly desired to develop drugs which are safe and highly effective in the treatment of these diseases.

Furthermore, many drugs have been developed for the treatment of malignant tumors. These anti-tumor drugs are roughly classified into the following two types. The first type includes so-called cytotoxins which directly suppress tumor growth. The second type includes those drugs which indirectly control the growth of tumors by recognizing them as foreign substances through the activation of the immunological protective functions of the host. However, drugs belonging to the former type do not have sufficient selective toxicity to tumor cells, and are toxic against normal cells of the host as well. Accordingly, their total dosage is limited considerably. On the other hand, the latter type, i.e. immunopotentiators, show unfavorable adverse reactions less frequently as compared with the former so that they are generally safely used. However, they have an essential problem in that since a tumor in itself is originated from normal cells of a patient, it may not be sufficiently recognized as a foreign substance by the immunological protective functions, and some immunopotentiators do not exhibit sufficient anti-tumor effect.

Disclosure of Invention

In view of the above, the present inventors have made an intensive investigation for a long period to develop a more effective therapeutic agent against allergic disorders, immune complex diseases and tumors. Consequently, they have found that human pepsin has a strong anti-allergic effect and a remarkable suppressive effect against various immune complex diseases, and at the same time shows an excellent anti-tumor effect. The present invention has been accomplished based upon the above finding.

Human pepsin is a known enzyme (Hirschowitz, Physiol. Rev., Vol. 37, p. 475, 1957). It can be obtained from human gastric juices, gastric mucous membranes, or urine by appropriately combining known methods for the purification of protein, for instance, salting-out, adsorption chromatography using an inorganic adsorbent, ion-exchange chromatography using an ion exchange resin, gel chromatography having molecular sieve effect, etc. A large quantity of human pepsin can also be obtained by cell culture of pepsin-producing cells, e.g., the chief cells of the gastric mucosa, pepsin-producing cells which are fused with cancer cells, or by genetic engineering wherein, for instance, a complementary-DNA is prepared by a reverse transcriptase from a template messenger RNA for human pepsin, and the DNA thus obtained is incorporated into E. coli.

For example, in accordance with the method of Tang (Methods in Enzymol., Vol. 19, p. 406, 1970), human gastric juice was passed through an Ambarlite IRC-50 column equilibrated with 0.2 M citrate-buffer solution (pH 3.0) to adsorb the pepsin thereon. The column was washed with 0.2 M citrate buffer solution (pH 3.8), and the pepsin was eluted with 0.2 M citrate buffer solution (pH 4.2). The eluate was repeatedly subjected to the chromatography in the same manner as above, thereby producing human pepsin.

The human pepsin obtained by the above method has a molecular weight of 32,000 to 38,000 by gel chromatography on Sephadex G-100; an isoelectric point of 1-3 by isoelectrofocusing on Ampholite; a maximum absorption at 278 nm; shows a positive reaction to the ninhydrin reaction; and is readily soluble in water and insoluble in ether or chloroform.

Human pepsin exhibits a high hydrolytic activity against hemoglobin in the acidic range of less than pH 7.0, while its activity is considerably inhibited by pepstatine. Meanwhile, human pepsin is stable in the acidic range of less than pH 7.0, while it is unstable in the alkaline range of more than pH 8.0.

The pharmacological action and toxicity of human pepsin will now be described with reference to experimental examples.

Experimental Example 1: Suppressive effect on production of anti-ovalbumin Ig E antibody

Groups each consisting of 10 Wistar strain male rats each weighing 180 to 200 g were used. One-tenth mg of ovalbumin together with 20 mg of aluminum hydroxide gel was injected intraperitoneally. From the next day forward, human pepsin was injected intravenously once a day for 14 days. After 7, 10 and 14 days from the administration of ovalbumin, blood samples were collected and measured for the level of anti-ovalbumin Ig E antibody in the serum by the homologous PCA reaction of rats (H. Maruyama, et al., Folia Pharmacologica Japonica, 74, 179, 1978). The results are shown in Fig. 1.

Production of anti-ovalbumin Ig E antibody was significantly suppressed by administration of human pepsin.

Experimental Example 2: Suppressive effect on bronchial asthma

Groups each consisting of 10 Wistar strain male rats each weighing 180 to 200 g were used. One-tenth mg of ovalbumin together with 20 mg of aluminum hydroxide gel was injected intraperitoneally and, from the next day on, human pepsin was injected intravenously once a day for 14 days. After 14 days, 25 mg/kg of ovalbumin was administered intravenously to induce bronchial asthma, and the resulting tracheal contraction was measured according to the method of Konzett and Rossler (Arch. Exptl. Path. Pharmacol. 195, 71, 1940). The relative contraction rate of the trachea of each group was calculated when the contraction of the control group was taken as 100. The results are shown in Table 1.

Table 1

| | | Contraction rate of trachea (%) |
|---|---|---|
| Control | | 100 |
| Human pepsin | 0.05 mg/kg | 75 |
| | 0.5 mg/kg | 48 |
| | 5 mg/kg | 25 |

The tracheal contraction was suppressed significantly by the administration of human pepsin.

Experimental Example 3: Suppressive effect on Masugi nephritis According to the method of Suzuki et al. (Folia Pharmacologica Japonica, 68, 572, 1972), rabbit anti-rat kidney serum was administered intravenously to groups each consisting of 10 Wistar strain male rats at a dose of 5 ml/kg to induce nephritis. After the occurrence of nephritis, blood and urine samples were taken at regular intervals to measure the levels of serum immune complex and urinary protein. Human pepsin was injected intravenously once a day after the administration of the anti-kidney antibody, and the control group was similarly administered inactivated human pepsin. The results are shown in Figs. 2 and 3.

In the groups treated with human pepsin, decreases in urinary protein and blood immune complex were observed.

Experimental Example 4: Suppressive effect on spontaneous kidney disorder

Measurement was made according to the method of Abe et al. (The Ryumachi, 14, 43, 1974).

Groups each consisting of sixteen 16-week old female mice (NZB x NZW) $F_1$, were injected with human pepsin intravenously at a dose of 10 mg/kg once a day. A control group was similarly administered inactivated human pepsin intravenously. Every fourth week, the urinary protein of the mice was measured by a commercial test paper (Combisticks). The results are shown in Fig. 4. Furthermore, 6 mice from each group were sacrificed at the age of 32 weeks and the cell infiltration into renal glomeruli was observed. The remaining 10 mice of each group received continued daily administration, and the survival rate at the age of 50 weeks was determined. The results are shown in Table 2.

It was observed that by the administration of human pepsin, the increase of urinary protein was significantly suppressed, the cell infiltration at 32 weeks was slight and the survival rate at 50 weeks was higher in the groups treated with human pepsin. These results indicate that spontaneous kidney disorder in mice was suppressed by human pepsin.

Table 2

| | Control group | Group treated with human pepsin |
|---|---|---|
| Cell infiltration | heavy infiltration in microlymphocytes and plasmocytes | slight infiltration around vessel wall |
| Survival rate | 20% | 80%* |

* $p < 0.05$

Experimental Example 5: Suppressive effect on thyroiditis

This experiment was carried out according to the method of Kotani et al. (Clinical Immunology, 9, (8), 635, 1977). Groups each consisting of 10 BUF/HDK male rats (6 weeks old) were subjected to thymectomy and thereafter, they were exposed to X-ray irradiations each of 200 rads repeated four times at an interval of two weeks. After 14 weeks from the thymectomy, the rats were sacrificed to exsanguinate. The thyroid glands were isolated and embedded in a paraffin block and then stained with hematoxylin-eosin or with azan. The severity of the thyroiditis was estimated according to the grades of 0 to 4 on the basis of the infiltration of mononuclear cells, destruction of endoplasmic reticulum and glandular fibrosis. Human pepsin was administered to the animals intravenously once a day, and the control group was similarly administered inactivated human pepsin. The results are shown in Table 3.

As compared with the control group, in the groups treated with human pepsin both the occurrence and the severity of thyroiditis were decreased in a dose dependent manner.

Table 3

| | Occurrence (%) | Severity |
|---|---|---|
| Control | 90 | $3.5 \pm 0.4$ |
| Human pepsin | | |
| 1 mg/kg | 80 | $2.9 \pm 0.5$ |
| 3 mg/kg | 60 | $2.0 \pm 0.2^{*}$ |
| 10 mg/kg | $40^{*}$ | $1.2 \pm 0.1^{**}$ |

$^{*}$ $p < 0.05$ $^{**}$ $p < 0.01$

Experimental Example 6: Hydrolysis of human immune complex

Serum was obtained from patients with rheumatoid arthritis, systemic lupus erythematosus and hepatitis who had been proved to carry immune complex in their serum. Human pepsin was added to the 1 ml of the serum in an amount of 10, 30 or 100 μg, and the serum was incubated at 37°C for 60 minutes. After the reaction, the amount of immune complex in the serum was measured by the hemolytic reaction of sheep erythrocytes in the presence of guinea pig complement according to the method of Fust et al. (Atherosclerosis, 29, 181, 1978) wherein aggregated human Ig G was used as a standard substance. The results are shown in Table 4.

Table 4

| Diseases | Serum No. | Amount of human pepsin added (μg/mℓ) | Immune complex content (μg/mℓ) |
|---|---|---|---|
| Rheumatoid arthritis | 1 | 0 | 75 |
| | | 10 | 66 |
| | | 30 | 52 |
| | | 100 | below 50 |
| | 2 | 0 | 234 |
| | | 10 | 181 |
| | | 30 | 153 |
| | | 100 | 122 |
| Systemic lupus erythematosus | 1 | 0 | 426 |
| | | 10 | 360 |
| | | 30 | 213 |
| | | 100 | 128 |
| | 2 | 0 | 120 |
| | | 10 | 69 |
| | | 30 | 58 |
| | | 100 | below 50 |
| Hepatitis | 1 | 0 | 63 |
| | | 10 | 58 |
| | | 30 | below 50 |
| | | 100 | below 50 |
| | 2 | 0 | 72 |
| | | 10 | 66 |
| | | 30 | 58 |
| | | 100 | 52 |

Human pepsin reduced the amount of immune complex in the serum of the patients suffering from chronic rheumatism, systemic lupus erythematosus and hepatitis in a dose dependent manner.

Experimental Example 7: Effect on human breast cancer MX-1 transplanted to a nude mouse

Two mm-square pieces of human breast cancer MX-1, which had been subcultured on nude mice (BALB/C, nu/nu), was transplanted subcutaneously on the backs of animals in groups each consisting of 5 nude mice of the said strain. From two weeks later, human pepsin was administered intravenously twice a day for 18 days. After 18 days from the first administration of human pepsin, the tumors were isolated and weighed. The results are shown in Table 5.

Table 5

| | Dose (mg/kg) | Weight of tumor (g) |
|---|---|---|
| Control | | $1.32 \pm 0.09$ |
| Human pepsin | 0.3 | $0.78 \pm 0.11^{*}$ |
| | 3.0 | $0.68 \pm 0.15^{*}$ |

* $p < 0.05$

Human pepsin showed a significant anti-tumor effect even at the lower dose.

Experimental Example 8: Acute toxicity

Groups each consisting of 10 ddY male mice (weighing 20 $\pm$ 1 g) were administered intravenously or intraperitoneally at a dose of 2 g/kg with human pepsin dissolved in physiological saline solution. Then, the mice were kept under daily observation for any toxicological symptoms for a week. No sign of any toxicity was observed throughout the period.

As described in the above Experimental Examples, human pepsin which is an effective ingredient of the pharmaceutical agent of the present invention suppressed production of Ig E antibody and showed a clearly therapeutic effect against experimental asthma. Morever, the enzyme clearly suppressed the establishment and development of a number of diseases which are believed to be induced by immune complexes, for example, thyroiditis and nephritis. Still further, this human pepsin exhibited a strong anti-tumor effect.

Judging from the results of the acute toxicity study, the dosage required to obtain these effects is within a sufficiently safe range. It is considered that because this human pepsin is a protein of human origin, it has little danger of inducing serious adverse reactions due to antigenicity, such as anaphylactic shock, and therefore it can provide a highly useful therapeutic agent against various allergic disorders including bronchial asthma, various immune complex diseases and various tumors.

## Brief Description of Drawings

Fig. 1 is a graph showing the results of Experimental Example 1.

Fig. 2 and Fig. 3 are graphs showing the results of Experimental Example 3.

Fig. 4 is a graph showing the results of the measurement of urinary protein in Experimental Example 4, wherein the content of urinary protein was measured on the basis of the coloration of the test paper and expressed in terms of an average of indices of a group. This index system consists of the numerals 0, 1, 2, 3 and 4, respectively corresponding to the colorations of (-), (+), (++), (+++) and (++++).

## Best Mode for Carrying Out the Invention

The therapeutic agent of the present invention is generally prepared in the form of an injectable solution and is injected intravenously, subcutaneously, intramuscularly or intra-articularly or to the local site of a tumor itself. However, it can be used also in the form of an oral agent, inhalant or rectal suppository. The daily dose of human pepsin is 1 to 1,000 mg, preferably 50 to 500 mg, but it can be suitably increased or decreased depending on age, symptoms and the manner of application.

Human pepsin can be formulated to a pharmacological agent by a conventional method together with any conventional pharmaceutical carriers or excipients.

Preparations for injection may include lyophilized preparations which are dissolved at the time of administration and liquid preparations; controlled release preparations are also preferred to keep a prolonged effective concentration.

Oral preparations may be in the form of capsules, tablets, granules, powders or liquid oral preparations, but they are preferably in the form of liposome inclusion bodies for the purpose of promoting absorption.

The inhalants are preferably in the form of lyophilized preparations; for rectal administration, the suppository form is preferred.

## Examples

Example 1

100 mg of human pepsin was dissolved in 10 mℓ of physiological saline solution and filtered aseptically through a membrane filter. Ten milliliter portions of the filtrate were placed in sterilized glass vessels and sealed after lyophilization to obtain lyophilized powder preparations.

Example 2

100 g of lyophilized human pepsin, 97 g of lactose and 3 g of magnesium stearate were each weighed and mixed uniformly. Then, 200 mg portions of this mixture were packed into No. 2 gelatin capsules and provided with an enteric coating to obtain enteric capsules.

Example 3

Yolk lecithin, cholesterol and diacetyl phosphate are mixed in a molar ratio of 7 : 2 : 1, and 100 mg of the mixture was dissolved in 12.5 mℓ of chloroform. From this solution a thin film was formed on the wall of a flask. A dispersion was prepared by mixing this film with 25 mℓ of phosphate buffer solution containing 100 mg of human pepsin. After ultrasonic treatment, the dispersion was centrifuged at 110,000 g. The resulting precipitate was suspended in 3 mℓ of physiological saline solution and sterilized to obtain a human pepsin containing liposome inclusion preparation.

WHAT IS CLAIMED IS:

1. A therapeutic agent for treatment of allergic disorders, immune complex diseases and tumors which contains human pepsin as an effective ingredient.

2. A therapeutic agent according to claim 1, containing an excipient and/or additive in addition to human pepsin.

3. A therapeutic agent according to claim 1 or 2, which is in a form of preparation for injection, oral preparation, rectal preparation, or preparation for inhalation.

FIG.1

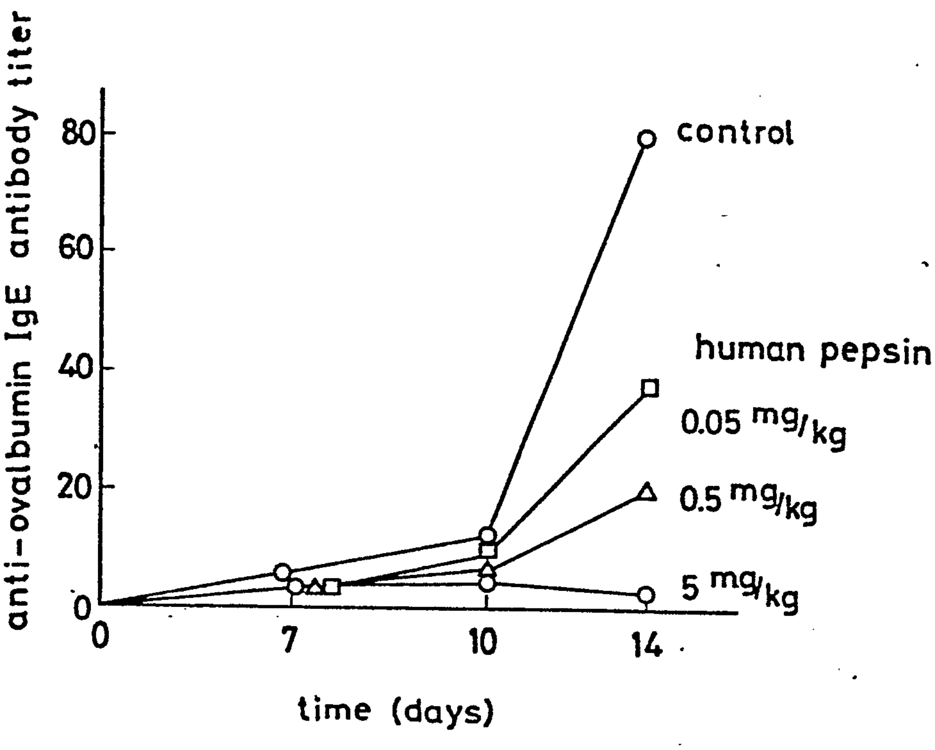
anti-ovalbumin IgE antibody titer
80
60
40
20
0
0
7
10
14
time (days)
control
human pepsin
0.05 mg/kg
0.5 mg/kg
5 mg/kg

FIG. 2

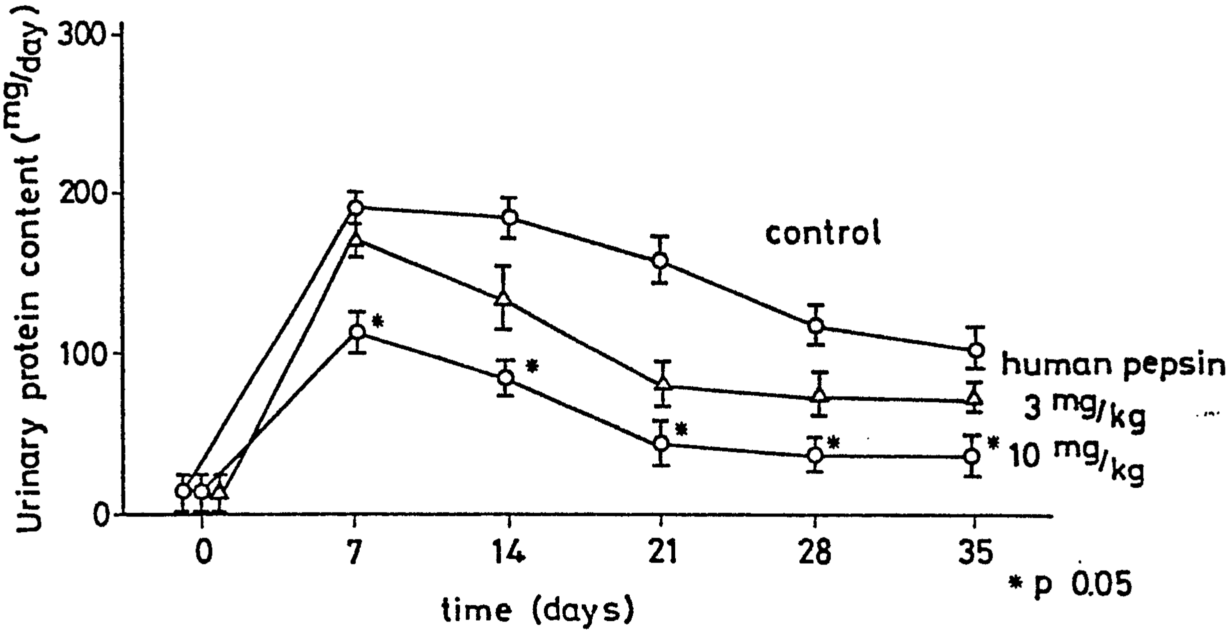
Urinary protein content (mg/day)
300
200
100
0
control
human pepsin
3 mg/kg
10 mg/kg
0
7
14
21
28
35
* p 0.05
time (days)

FIG. 3

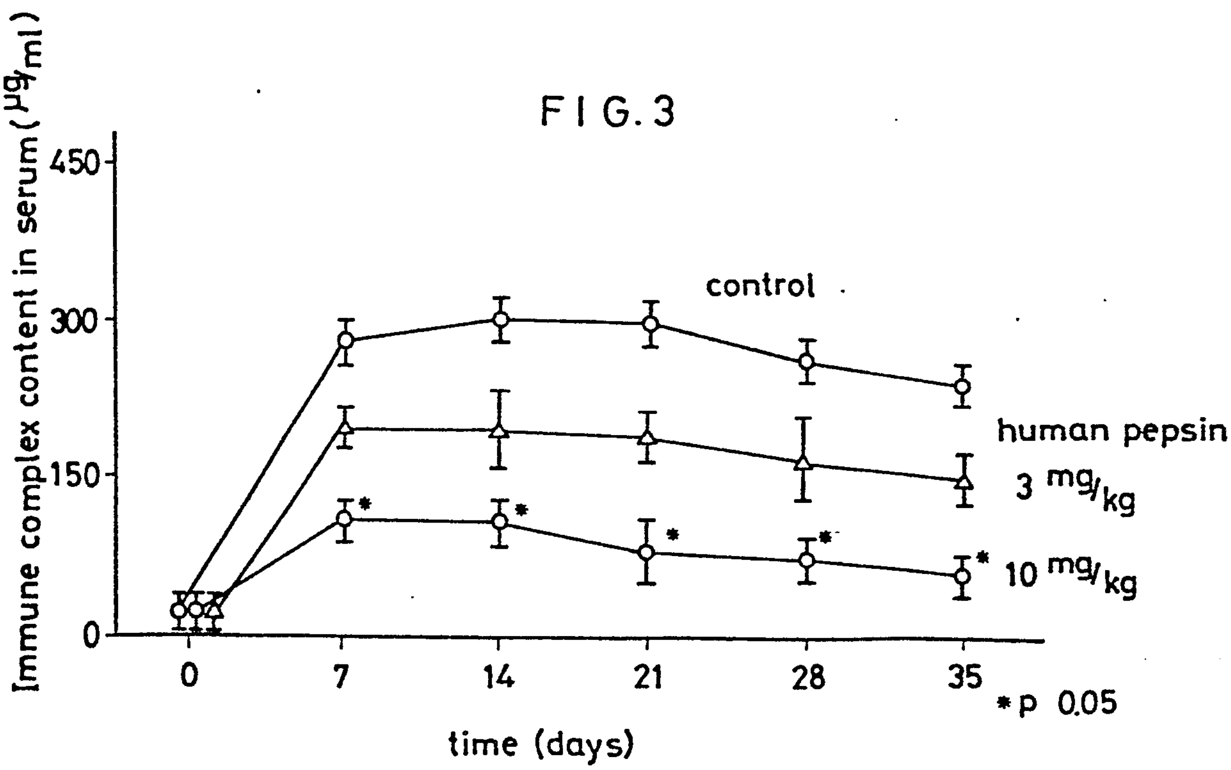
Immune complex content in serum (μg/ml)
450
300
150
0
control
human pepsin
3 mg/kg
10 mg/kg
0
7
14
21
28
35
* p 0.05
time (days)

FIG.4

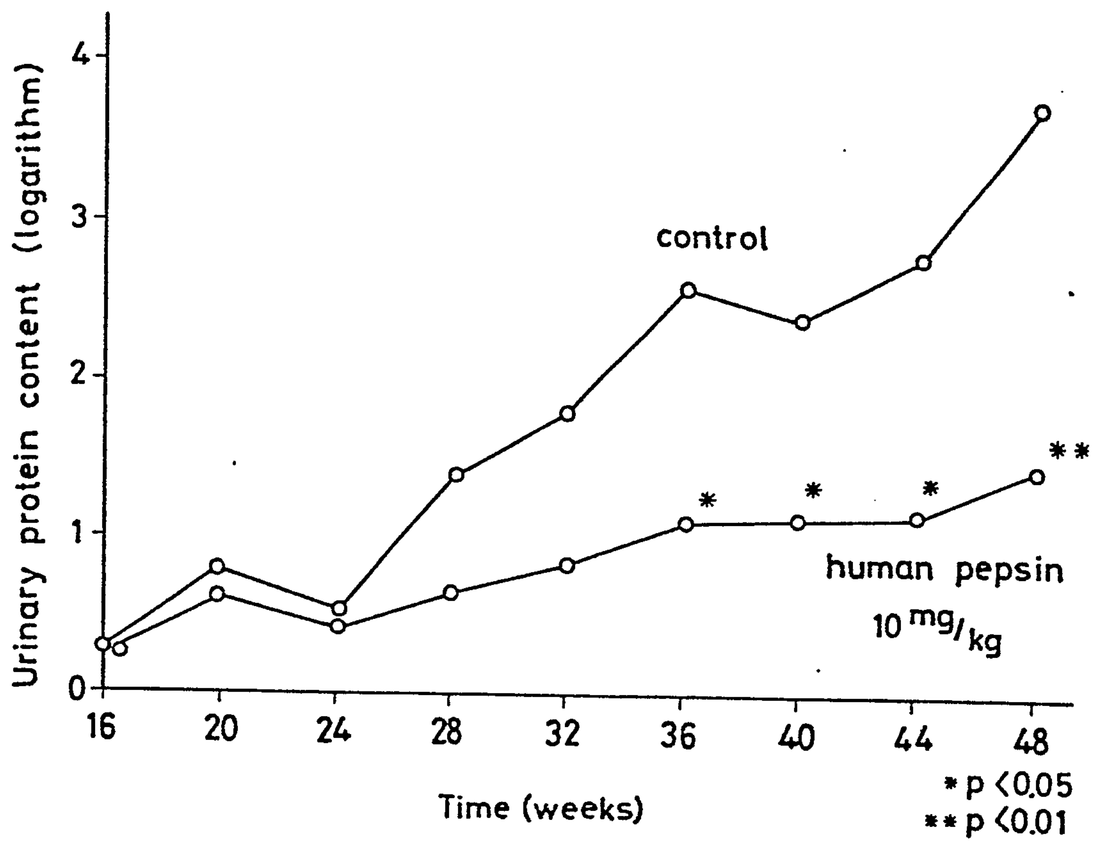

4
3
2
1
0
Urinary protein content (logarithm)
control
human pepsin
10 mg/kg
* * * **
16
20
24
28
32
36
40
44
48
Time (weeks)
* p <0.05
** p <0.01

# INTERNATIONAL SEARCH REPORT

**0100366**

International Application No PCT/JP82/00037

**I. CLASSIFICATION OF SUBJECT MATTER** (if several classification symbols apply, indicate all) [3]

According to International Patent Classification (IPC) or to both National Classification and IPC

Int. Cl.[3] A61K 37/54

**II FIELDS SEARCHED**

Minimum Documentation Searched [4]

| Classification System | Classification Symbols |
|---|---|
| I P C | A61K 37/54 |

Documentation Searched other than Minimum Documentation to the Extent that such Documents are Included in the Fields Searched [5]

**III. DOCUMENTS CONSIDERED TO BE RELEVANT** [14]

| Category * | Citation of Document, [16] with indication, where appropriate, of the relevant passages [17] | Relevant to Claim No. [18] |
|---|---|---|
| | | |

* Special categories of cited documents: [15]

"A" document defining the general state of the art

"E" earlier document but published on or after the international filing date

"L" document cited for special reason other than those referred to in the other categories

"O" document referring to an oral disclosure, use, exhibition or other means

"P" document published prior to the international filing date but on or after the priority date claimed

"T" later document published on or after the international filing date or priority date and not in conflict with the application, but cited to understand the principle or theory underlying the invention

"X" document of particular relevance

**IV. CERTIFICATION**

| Date of the Actual Completion of the International Search [2] | Date of Mailing of this International Search Report [2] |
|---|---|
| April 22, 1982 (22.04.82) | May 10, 1982 (10.05.82) |
| International Searching Authority [1] | Signature of Authorized Officer [20] |
| Japanese Patent Office | |